# EUROPÄISCHE PATENTSCHRIFT

(11) **EP 2 482 750 B1**
(45) Veröffentlichungstag und Bekanntmachung des Hinweises auf die Patenterteilung: **20.05.2015**
(21) Anmeldenummer: 10732650.6
(22) Anmeldetag: 28.06.2010
(51) Int. Cl.: A61B 19/08, A61B 19/00

(54) **CHIRURGISCHES ABDECKTUCH**
SURGICAL DRAPE
DRAP CHIRURGICAL

(30) Priorität: 30.09.2009 DE 102009047896
(43) Veröffentlichungstag der Anmeldung: 08.08.2012
(73) Patentinhaber: Paul Hartmann Aktiengesellschaft, 89522 Heidenheim (DE)
(72) Erfinder: STANG, Silke, 89522 Heidenheim (DE)
(74) Vertreter: DREISS Patentanwälte PartG mbB
(86) Internationale Anmeldenummer: PCT/EP2010/003928
(87) Internationale Veröffentlichungsnummer: WO 2011/038792

(56) Entgegenhaltungen:
- EP-A1- 1 641 407
- WO-A1-96/38096
- US-A1- 2006 169 290
- US-A1- 2007 175 486

## Beschreibung

Die Erfindung betrifft ein chirurgisches Abdecktuch umfassend ein flächiges, flexibles Abdeckelement mit einem Bereich zur Aufnahme eines aus der Ebene des Abdeckelements herausragenden Referenzmittels für ein Navigationssystem.

Chirurgische Abdecktücher dienen dazu, den Bereich eines chirurgischen Eingriffs sauber und steril zu halten. Abdecktücher weisen hierzu Öffnungen im Eingriffsbereich auf, wobei in diesen Bereichen sogenannte Inzisionsfolien vorgesehen sein können, so dass bis zum Eingriffszeitpunkt, wie beispielsweise eines Schnittes zur Öffnung des Körpers des Patienten der Operateur das Operationsfeld im Blick hat und die gesamte abgedeckte Fläche steril ist.

Darüber hinaus sind auch chirurgische Abdecktücher bekannt, bei denen im Bereich einer Öffnung ein Abdeckmittel für ein aus der Ebene der Oberfläche des Abdecktuches herausragendes Referenzmittel aufgenommen ist, welches für ein zwei- oder dreidimensionales Vermessungssystem identifizierbar ist.

Insbesondere bei neurologischen chirurgischen Eingriffen am menschlichen Körper ist es vielfach vorgesehen, den genauen chirurgischen Aktionsbereich vor dem Eingriff zu vermessen, um ihn so genau wie möglich zu lokalisieren und gesundes Gewebe zu schonen. Darüber hinaus ist es auch vorgesehen, während des Eingriffs selber die Ergebnisse intraoperativ mittels Computertomographen, aber auch anderen bildgebenden Verfahren, wie Röntgen oder Kernspin o. ä. zu kontrollieren.

Zur Beibehaltung der genauen Lage des chirurgischen Aktionsbereichs, der durch die vorherige Vermessung festgelegt wurde, werden während der Operation weitere bzw. andere Vermessungssysteme eingesetzt, die sowohl zwei- als auch dreidimensional arbeiten können, z. B. mit zwei Infrarotsendern und -empfängern. Durch den Wechsel der Koordinatenbezugspunkte für den chirurgischen Aktionsbereich beim Wechsel des Navigationssystems, das ein gleiches oder ein anderes sein kann, ist es daher zumeist vorgesehen, im Bereich des chirurgischen Eingriffs ein geeignetes Referenzmittel bereitzustellen, das durch das zweite Vermessungssystem identifiziert wird.

So werden z. B. häufig zwei- oder dreidimensionale Navigationssysteme mit der entsprechenden Anzahl von z. B. Infrarotsendern und -empfängern eingesetzt, welche ein Referenzmittel z. B. in Form eines dreiarmigen Sterns (auch Referenzstern genannt) umfassen, wobei der Referenzstern auch als Navigationsbasiseinheit beschrieben sein kann.

Es ergibt sich dabei vielfach die Notwendigkeit, das Referenzmittel möglichst steril, aber für das Navigationssystem identifizierbar, anzuordnen. Dabei ragt das Referenzmittel vielfach aus der Ebene, die durch das Abdecktuch überdeckt ist, heraus und liegt meist oberhalb des Eingriffsbereichs.

Ein derartiges Navigationssystem ist beispielsweise in der EP 1 923 015 A1 beschrieben, wobei die entsprechenden Referenzmittel beispielsweise an Gegenständen, insbesondere an medizinischen Instrumenten oder an Körperstrukturen angebracht werden können.

Das aus der EP 1 641 407 B1 bekannte Abdecktuch beschreibt dabei einen weiteren Fenestrationsbereich neben dem Eingriffsbereich, in dem eine bereits vorgeformte haubenartige Folie einsetzbar ist, wobei im Bereich der haubenartigen Folie dann das Referenzmittel eingesetzt werden kann. Ferner sind sogenannte Verjüngungsmittel beschrieben, mittels derer die Haube dann an die Form des Referenzmittels angepasst werden kann, indem sie durch Zusammenziehen der Verjüngungsmittel so in ihrem Volumen verringert wird, dass sie vorzugsweise faltenfrei gegen die Struktur des Referenzmittels anliegt. Ebenso sind Längliche, an dem Abdecktuch fixierbare Verkürzungsmittel vorgesehen, mittels derer das Abdecktuch verkürzt werden kann.

Nachteilig an dem beschriebenen Abdecktuch ist dabei, dass auch im nicht verwendeten Zustand der haubenartige, aus der Ebene des Tuchs hervorragende Teil, der durch ein Abdeckmittel gebildet ist, welches mit dem Abdecktuch am Umfang einer zweiten Fenestration verbunden ist, vorgesehen ist, oder die beiden Elemente, nämlich Abdecktuch und Abdeckmittel werden separat aufbewahrt und müssten separat sterilisiert werden und werden dann erst zur Operation miteinander verbunden. Sind die beiden Elemente jedoch bereits miteinander verbunden, so ergibt sich eine erschwerte Lagerung des Abdecktuchs aufgrund des aus der Ebene des flächigen Abdecktuchs herausragenden Abdeckmittels, das ähnlich einer Glocke aus dem Abdecktuch hervorsteht.

Der Erfindung stellt sich nun die Aufgabe, diese Nachteile zu vermeiden und ein chirurgisches Abdecktuch, insbesondere für chirurgisch neurologische Verfahren bereitzustellen, das einen Bereich zur Aufnahme eines aus der Ebene des Abdeckelements herausragenden Referenzmittels für ein Navigationssystem bereitstellt, und auf der anderen Seite eine einfache Lagerung und Aufbewahrung des Abdecktuchs, insbesondere eine möglichst falten- und knickfreie Lagerung des für die Aufnahme des Referenzmittels vorgesehenen Bereiches des Abdecktuches ermöglicht. Zudem stellt sich die Erfindung die Aufgabe, einen für den jeweiligen Einsatzbereich individuell einstellbaren Bereich zur Aufnahme von aus der Ebene des Abdeckelements herausragenden Referenzmittel bereitzustellen.

Die Erfindung löst diese Aufgabe durch ein chirurgisches Abdecktuch umfassend ein flächiges, flexibles Abdeckelement mit einem Bereich zur Aufnahme eines aus der Ebene des Abdeckelementes herausragenden Referenzmittels für ein Navigationssystem, wobei der Bereich mindestens zwei verformbare, längliche mit dem Abdeckelement zumindest partiell verbundene zwei Stabenden aufweisende Stabelemente umfasst, welche den Bereich zur Aufnahme des Referenzmittels zwischen sich einschließen, sowie mindestens ein an dem Abdeckelement fixierbares mit zumindest einem der Stabelemente zusammenwirkendes längliches Verkürzungsmittel zur Verringerung und/oder Fixierung des Abstandes der zwei Stabenden zueinander in der Ebene des Abdeckelementes, so dass der Bereich zur Aufnahme des Referenzmittels sich aus der Ebene des Abdeckelements erhebt.

Besonders vorteilhaft bei einer solchen Ausgestaltung ist, dass bei dem chirurgischen Abdecktuch, das ein flächiges flexibles Abdeckelement umfasst, nämlich das eigentliche Tuchmaterial zur Abdeckung des Körpers eines Patienten, mit einem Bereich, in dem dann, nachdem er sich über die Ebene der Oberfläche des Abdeckelements erhebt, das Referenzmittel eines Navigationssystems untergebracht werden kann, im Lagerungszustand, also in einem Zustand, in dem es nicht verwendet wird, der Bereich, in dem das Referenzmittel aufgenommen werden kann, sich ebenfalls in der Ebene des flexiblen flächigen Abdeckelements befindet. D. h. es handelt sich bei dem chirurgischen Abdecktuch im unbenutzten bzw. unverformten Zustand der Stabelemente um eine vollständig flächige flexible Materialbahn bestehend aus einem Abdeckelement mit einem in der gleichen Ebene liegenden Bereich, in dem dann später das Referenzmittel aufgenommen werden kann.

Durch die verformung der mindestens zwei Stabelemente, so dass der Abstand ihrer jeweiligen Stabenden zueinander verringert wird, wird ein tunnelartiger Bereich zwischen den beiden bogenförmig verformten Stabelementen bereitgestellt, in den dann das Referenzmittel aufgenommen werden kann. Besonders vorteilhaft ist hierbei, dass durch die tunnelartige Form eine besonders glatte Haube bereitgestellt werden kann, die im Bereich des Referenzmittels weder Nähte noch Knicke aufweist, so dass beispielsweise die Scanfunktion einer Kamera des Navigationssystems vorteilhaft ohne Reflexionen erfolgen kann.

An diesem Abdeckelement, welches jegliche Form, wie insbesondere quadratische, rechteckige, dreieckige, ovale, runde, vor allem insbesondere eine rechteckige Form aufweisen kann, sind mindestens zwei Stabelemente vorgesehen, die verformbar gestaltet sind. Die Stabelemente können dabei entweder elastisch verformbar oder insbesondere bevorzugt plastisch verformbar sein. Plastisch verformbare Stabelemente finden nicht wieder oder im Wesentlichen nicht wieder in ihre ursprüngliche Form zurück, sobald die Kraft, die die Verformung bewirkt, aufgehoben wird.

Die Verbindung zwischen den Stabelementen und dem Abdeckelement ist dabei zumindest partiell, wobei insbesondere eine über die gesamte Länge des Stabelementes bestehende Fixierung mit dem Abdeckelement bevorzugt ist. Generell ist es jedoch auch möglich, die Stabelemente mit dem Abdeckelement beispielsweise im Bereich der zwei Stabenden sowie in einem oder mehreren diskreten mittleren Bereichen zu verbinden. Insbesondere kann vorgesehen sein, dass die Stabelemente über eine darauf aufgebrachte Kunststoffbeschichtung mit dem Abdeckelement verbunden werden. Alternativ können die Stabelemente mittels Klebemittel auf dem Abdecktuch fixiert werden. Alternativ können die Stabelemente mit dem Abdecktuch verschweißt werden. Alternativ können die Stabelemente auch in Taschen am Abdeckelement geführt sein.

Die länglichen Verkürzungsmittel dienen insbesondere zur Ausbildung einer im Wesentlichen in der Ebene des Abdeckelements liegenden Begrenzung des Bereiches des Abdeckelements, welcher sich aus der Ebene des Abdeckelements erhebt.

Die länglichen Verkürzungsmittel zur Verringerung und/oder Fixierung des Abstandes der zwei Stabenden zueinander in der Ebene des Abdeckelementes können insbesondere durch ein textiles Bandelement gebildet werden, aber auch durch einen Streifen, beispielsweise einer Klebefolie. Dabei kann vorgesehen sein, dass das Verkürzungsmittel über seine gesamte Länge oder lediglich in einzelnen Bereichen, insbesondere an den beiden Endbereichen des länglichen Verkürzungsmittels, haftend ausgebildet ist. Grundsätzlich kann sowohl eine Haftung über ein kohäsives als auch adhäsives Material in Frage kommen, oder grundsätzlich können auch Hakenhaft- und Ösenhaftmaterialien vorgesehen sein.

So kann insbesondere vorgesehen sein, dass das Verkürzungsmittel bei einer Lagerung und vor der Anwendung des chirurgischen Abdecktuchs mit seinem einen Ende bereits mit dem Abdeckelement lösbar oder unlösbar verbunden ist und der andere Endbereich entweder unverbunden mit dem Abdeckelement vorgesehen ist oder aber in einer Position mit dem flexiblen Abdeckelement verbunden ist, in der die Stabelemente noch nicht verformt sind. Es kann dann insbesondere vorgesehen sein, dass nach Einwirkung von Verformungskräften auf das Stabelement zur Verformung des Stabelements und dessen Hervorhebung aus der Ebene des Abdeckelements, insbesondere bei plastisch verformbarem Stabelement, durch die Fixierung des länglichen Verkürzungsmittels an einem zweiten Haftelement die hervorgerufene Verformung des Stabelements weiter stabilisiert wird. Alternativ kann auch vorgesehen sein, dass durch Fixierung des länglichen Verkürzungsmittels an einem zweiten Haftelement eine Verformung des insbesondere elastisch verformbaren Stabelements hervorgerufen wird, wodurch das Stabelement gebogen wird und sich aus der Ebene des Abdeckelements erhebt. Generell wird durch die Verformung der Stabelemente dann eine Haube gebildet, also ein Hohlraum, und zwar derart, dass aufgrund der Verbindung von Stabelement und Abdeckelement das Abdeckelement in diesem Bereich zusammen mit dem Stabelement aus der ursprünglichen Ebene des Abdeckelements hervorgehoben wird. In diesen Hohlraum unterhalb des Bereichs des Abdeckelements kann dann das Referenzmittel aufgenommen werden.

Dabei kann vorgesehen sein, dass das Verkürzungsmittel erste Haftmittel aufweist, die mit zweiten Haftmitteln am Abdeckelement zusammenwirken. Dabei können die zweiten Haftmittel am Abdeckelement auch durch das Abdeckelement selbst gebildet werden, z. B. sofern es sich bei dem Abdeckelement um ein Folienelement handelt und das Verkürzungsmittel selber ein klebendes bandförmiges Element ist oder klebende Bereiche enthält. Zusätzliche separate zweite Haftmittel sind dann nicht notwendig. Die ersten Haftmittel können dann adhäsive Haftmittel, beispielsweise eine klebende Beschichtung auf einer Seite des Verkürzungsmittels, sein. Alternativ können auch separate zweite Haftmittel am Abdeckelement angebracht sein, insbesondere in Form von Klebeelementen oder Ösenhaft- bzw. Hakenhaftelementen. Diese Ösenhaft- bzw. Hakenhaftelemente können dabei mit korrespondierenden Haken- bzw. Ösenhaftelementen als erste Haftmittel des Verkürzungselements zusammenwirken.

Haken-/Ösenhaftelemente werden oftmals auch als Hook-/Loop-Elemente oder Kletthaken/Klettflausch-Elemente oder Haken/Schlaufen-Elemente bezeichnet.

Dabei kann beispielsweise vorgesehen sein, dass das Verkürzungsmittel über seine gesamte Länge mit ersten Haftmitteln versehen ist. Damit kann die Fixierung des Verkürzungselements an dem zweiten Haftelement vorteilhaft in beliebigen Schritten erfolgen, je nachdem an welchem Punkt des Verkürzungsmittels dieses mit den zweiten Haftelementen verbunden wird.

Besonders bevorzugt besteht das Verkürzungselement aus einem textilen ein- oder zweiseitigen Flauschmaterial, so dass hierdurch ein Ösenhaftelement als erstes Haftmittel gebildet wird, und insbesondere kann hierbei ein streifen- oder bandförmiges Material eingesetzt werden, das mit zwei oder mehr Hakenhaftelementen als zweite Haftmittel, die insbesondere in Form von rechteckigen oder quadratischen Elementen auf dem Abdecktuch angebracht sind, verbindbar ist. Die entsprechenden zweiten Haftelemente in Form von Hakenhaftelementen sind dabei vorzugsweise im Bereich der Stabenden bei unverformten Stäben angeordnet. Insbesondere bei elastisch verformbaren Stabelementen kann damit vorteilhaft durch die stufenlose Verkürzung des Abstandes zwischen den zwei Hakenelementen durch das über die gesamte Länge als Flauschmaterial ausgebildete Verkürzungsmittel die Verformung der Stabelemente individuell eingestellt werden.

Alternativ können erste Haftmittel auch nur abschnittsweise auf dem Verkürzungsmittel, insbesondere vorzugsweise auch nur in den Endbereichen des Verkürzungsmittels vorgesehen sein. Generell gilt, je stärker die Verformung und damit die Aufwölbung der Stabelemente, umso höher wird der dadurch gebildete Bereich aus der Ebene herausgehoben. Gleichzeitig wird damit die Ausdehnung des Bereichs in der Ebene verringert.

Es kann vorgesehen sein, dass zumindest eines der Enden des oder der Verkürzungsmittel lösbar mit dem Abdeckelement, insbesondere mit seinem ersten Haftmittel mit dem zweiten Haftmittel am Abdeckelement, verbunden ist. Es kann insbesondere vorgesehen sein, dass das Verkürzungsmittel mit beiden Endbereichen lösbar mit dem Abdeckelement verbunden ist. Insbesondere ist das Verkürzungsmittel an seinen beiden Enden mit seinem ersten Haftelement lösbar mit dem zweiten Haftelement am Abdeckelement verbunden. Weiter alternativ kann das Verkürzungsmittel mit einem Endbereich fest und unlösbar verbunden mit dem Abdeckelement vorgesehen sein und lediglich an seinem zweiten Endbereich oder über seine restliche oder teilweise Länge erste Haftmittel tragen.

In einer alternativen Ausführungsform können das/die Verkürzungsmittel noch unbefestigt dem Abdeckelement zur individuell bedarfsgerechten Festlegung an dem/den zweiten Haftelement/en beigelegt sein.

Vorzugsweise können mindestens zwei zweite Haftmittel je Verkürzungsmittel vorgesehen sein. Grundsätzlich sind auch drei oder mehr zweite Haftmittel denkbar.

Die zweiten Haftelemente sind vorzugsweise im Bereich der Stabenden am Abdeckelement vorgesehen. Vorzugsweise sind die zweiten Haftmittel im Bereich der Stabenden der Stabmittel an deren nach außen gerichteter Seite, also der den Seitenkanten des Abdecktuches zugewandter Seite, angeordnet.

Die zweiten Haftelemente sind am Abdeckelement vorzugsweise dauerhaft und unlösbar verbunden.

Es sind erfindungsgemäß zwei Stabelemente vorgesehen, die den Bereich zur Aufnahme des Referenzmittels zwischen sich einschließen, wobei insbesondere jedem der Stabelemente ein Verkürzungsmittel zugeordnet ist.

Es können vorteilhaft auch noch weitere Stabelemente vorgesehen sein, insbesondere vorzugsweise drei oder vier Stabelemente.

Weitere Stabelemente sind insbesondere im Falle der Ausbildung eines größer dimensionierten Bereiches, insbesondere mit einer größeren Längserstreckung, für die Stabilisierung dieses Bereiches, welcher sich aus der Ebene des Abdeckelements erhebt, vorteilhaft.

Im Falle von mehr als zwei Stabelementen ist vorteilhafterweise jedem der äußerst gelegenen Stabelementen, insbesondere an deren nach außen gerichteter Seite, also der den Seitenkanten des Abdecktuches zugewandter Seite ein Verkürzungsmittel zugeordnet.

Die Stabelemente sind vorzugsweise parallel zueinander angeordnet. Der Abstand der Stabelemente zueinander hängt von der Größe der benötigten Haube ab, also der Größe des Bereichs, der für ein aus der Ebene des Abdeckelements herausragendes Referenzmittel eines Navigationssystems benötigt wird. In dem Fall, dass mehr als zwei Stabelemente vorgesehen sind, können vorzugsweise alle Stabelemente parallel angeordnet sein. Insbesondere können in diesem Fall, aber auch wenn lediglich zwei Stabelemente vorgesehen sind, die Stabelemente verschiedene Längen aufweisen. Insbesondere bei mehr als zwei Stabelementen kann vorgesehen sein, dass die bei paralleler Anordnung entstehenden Stabelementengruppen, die zwischen sich den Bereich zur Aufnahme des Referenzmittels einschließen, so ausgebildet sind, dass die Stabelemente mit der größeren Längserstreckung innerhalb der kürzeren Stabelemente einer Gruppe angeordnet sind, da hierdurch die Stabilität des Bereichs zur Aufnahme des Referenzmittels verbessert wird.

Grundsätzlich können die Stabelemente jedoch auch abweichend von einer parallelen Anordnung vorgesehen sein, insbesondere können zwei Enden der Stabelemente zueinander einen geringeren Abstand aufweisen, als die beiden anderen Stabelemente, so dass der dazwischen entstehenden Bereich nicht wie bei der parallelen Anordnung rechteckig ist, sondern entweder dreieckig, bei einander berührenden Stabenden der zwei Stabelemente oder trapezförmig, wenn die Stabenden beabstandet sind.

Das Abdeckelement umfasst vorzugsweise zumindest teilweise transluzentes, insbesondere transparentes Material. Insbesondere der zur Aufnahme des Referenzmittel vorgesehene Bereich ist aus einem transluzenten, insbesondere transparenten Material gebildet. Weiter vorzugsweise kann das Abdeckelement vollständig aus einem transluzenten, insbesondere transparenten Material bestehen. Besonders bevorzugt ist dabei eine Ausgestaltung des Abdeckelements oder zumindest des Bereichs aus transluzenten, insbesondere transparenten Material als Folie.

Die Ausführung zumindest des zur Aufnahme des Referenzmittels vorgesehenen Bereiches in Form eines transluzentes, insbesondere transparentes Material, insbesondere in Form einer transluzenten, insbesondere einer transparenten Folie ist vorteilhaft, da das in diesem Bereich eingebrachte Referenzmittel vom medizinischen Personal direkt erkennbar ist, und damit die Kontrolle und damit auch die Sicherheit während eines Eingriffes am Patienten erhöht wird.

Weiter vorzugsweise ist das transluzente, insbesondere transparente Material, insbesondere die transluzente, insbesondere transparente Folie durchgängig für Infrarot mit einer Wellenlänge von 780 nm bis 1 mm, weiter insbesondere mit einer Wellenlänge von 780 nm bis 3000 nm und weiter insbesondere mit einer Wellenlänge von 780 nm bis 1400 nm.

Die Folie umfasst insbesondere polymere Materialien entnommen aus der Gruppe Polyethylen (PE), Polypropylen (PP), Polyamid (PA), Polyester (PET) oder Kombinationen davon. Die Folien können auch aus Mischungen und/oder Verbunden aus den vorgenannten Materialien bestehen. Insbesondere bevorzugt wird eine Folie aus einem Polypropylenmaterial und/oder Polyethylenmaterial eingesetzt.

Insbesondere wird eine Folie vorzugsweise mit einer Dicke von kleiner 200 µm, insbesondere kleiner 150 µm und insbesondere kleiner 100 µm eingesetzt.

Die Stabelemente sind erfindungsgemäß verformbar ausgestaltet. Die Stabelemente können Drahtelemente oder metallumfassende Elemente sein. Die Stabelemente können insbesondere Kunststoffstäbe oder kunststoffbeschichtete Stäbe sein.

Die Länge der Stabelemente beträgt vorzugsweise mindestens 20 cm, weiter vorzugsweise mindestens 30 cm, weiter vorzugsweise mindestens 40 cm, weiter vorzugsweise mindestens 50 cm, insbesondere vorzugsweise höchstens 100 cm, weiter vorzugsweise höchstens 90 cm, weiter vorzugsweise höchstens 80 cm.

Die Breite der Stabelemente beträgt vorzugsweise mindestens 0,5 cm, weiter vorzugsweise mindestens 0,8 cm, insbesondere höchstens 3,5 cm, weiter vorzugsweise höchstens 3,0 cm, weiter vorzugsweise höchstens 2,5 cm, weiter vorzugsweise höchstens 2,0 cm.

Bei der Anordnung der mindestens zwei Stabelementen beträgt der Abstand der direkt benachbarten Stabelemente vorzugsweise mindestens 10 cm, weiter vorzugsweise mindestens 20 cm, weiter vorzugsweise mindestens 25 cm, weiter vorzugsweise mindestens 30 cm, insbesondere vorzugsweise höchstens 80 cm, weiter vorzugsweise höchstens 60 cm, weiter vorzugsweise höchstens 50 cm, weiter vorzugsweise höchstens 40 cm.

Der Abstand zwischen den Stabelementen wird dabei innerhalb der zueinander gerichteten Kanten der jeweils direkt benachbarten Stabelemente im unverformten Zustand bzw. in den ursprünglich unverformten rückgängig gebrachten Zustand vermessen. Bei nicht paralleler Anordnung der Stabelemente liegt vorzugsweise sowohl der größere wie auch der kleinere Abstand in den genannten Grenzen. Sofern bei mehr als zwei Stabelementen zwei Gruppen aus Stabelementen gebildet werden, kann der Abstand zwischen den Gruppen vorzugsweise im vorstehend genannten Bereich liegen.

Durch diesen Abstand kann dann durch Verformen der Stabelemente vorteilhaft eine Haube bereitgestellt werden, unter welche ein Großteil der Referenzsysteme (Referenzmittel) eingebracht werden kann und wobei durch die zwischen den Stabelementen bereitgestellte Fläche eine für die Navigationsfunktion, wie beispielsweise Abscannen, hindernisfreie Fläche bereitgestellt wird, ohne dass es aber durch einen zu großen Abstand zu einem nicht stabilisierten und damit unvorteilhaften Durchhängen des Abdeckelements kommen würde.

Grundsätzlich ist es auch möglich, die Stabelemente mit Gelenken auszustatten, so dass durch Verringerung des Abstandes der Stabenden zueinander keine Verformung der Stabelemente über deren gesamte Länge in einer bogenförmigen Weise erfolgt, sondern die Stabelemente um ihre Gelenke abgewinkelt werden, so dass dann beispielsweise bei Vorhandensein von einem Gelenk pro Stabelement eine zeltförmige Haube oder beispielsweise bei Vorhandensein von zwei Gelenken pro Stabelement eine quaderförmige Haube gebildet wird.

Weiterhin kann auch vorgesehen sein, dass das Abdeckelement teilweise auch nicht folienartige Materialien umfasst bzw. aus einem nicht folienartigen Material gebildet ist, wobei diese nicht folienartigen Materialien insbesondere nicht transluzent oder nicht transparent ausgebildet sind. Für diese nicht folienartigen Materialien werden vorzugsweise Vliesmaterialien, insbesondere Spinnvliese und/oder Meltblown-Vliese, eingesetzt.

Vorzugsweise kann das Abdeckmaterial teilweise Vliesmaterialien oder Vlies-Folien-Laminate oder Vlies-Folien-Vlies-Laminate umfassen oder aus diesen teilweise gebildet sein. Dabei ist insbesondere vorgesehen, dass jedoch wenigstens der Bereich zur Aufnahme eines aus der Ebene des Abdeckelements herausragenden Referenzmittels für ein Navigationssystem aus einem transluzenten oder transparenten Material, insbesondere einer transluzenten oder transparenten Folie ausgebildet ist. Die zwei Materialien können dann beispielsweise über ein Schweißverfahren miteinander verbunden sein. Grundsätzlich sind jedoch auch andere Verbindungsverfahren, wie klebende Verfahren oder Nahtverfahren, denkbar.

Besonders bevorzugt ist jedoch die Ausbildung des gesamten Abdeckelements aus einem einheitlichen Folienmaterial.

Das chirurgische Abdecktuch ist vorteilhaft im Einsatz als ein neurochirurgisches Abdecktuch und wird als solches verwendet.

Unter Neurochirurgie wird dabei der chirurgische Eingriff im Bereich der Wirbelsäule und/oder Kopfbereich verstanden.

Das chirurgische Abdecktuch ist vorteilhaft auch in anderen Bereichen im Einsatz, in denen mit Referenzmitteln für Navigationssysteme gearbeitet wird. Denkbar ist beispielsweise auch der Einsatz im orthopädischen Bereich, beispielsweise beim Einbringen von Implantaten in Form von Endoprothesen.

Des Weiteren ist vorgesehen, dass eine Sterilisierung des Abdecktuchs vorgenommen wird, wobei dies in üblicher Weise, beispielsweise mittels einer Gammastrahlung, erfolgen kann. Alternativ kann jedoch auch eine Ethylenoxidsterilisierung oder ein anderes im Stand der Technik bekanntes verfahren zur Sterilisierung eingesetzt werden.

Das chirurgische Abdecktuch ist vorteilhafterweise ein bereits herstellerseitig steril dargebotenes Abdecktuch.

Insbesondere vorteilhaft wird das chirurgische Abdecktuch herstellerseitig in einem gefalteten Zustand angeboten.

Insbesondere vorteilhaft ist das chirurgische Abdecktuch derart gefaltet, dass der zwischen den mindestens zwei Stabelementen und dann für die Aufnahme von Referenzmitteln vorgesehene Bereiche weitgehend faltenfrei bleibt.

Im Falle von längeren, insbesondere plastisch verformbaren Stabelementen werden diese Stabelemente zum Zusammenlegen des chirurgischen Abdecktuchs vorteilhafterweise lediglich nur einmal geknickt und damit auf sich selbst umgelegt. Dadurch wird das Abdeckelement in dem für die Aufnahme von Referenzmittel vorgesehenen Bereich lediglich nur mit einer Falte belastet.

Insbesondere vorteilhaft wird das chirurgische Abdecktuch herstellerseitig steril verpackt dargeboten.

Weitere Vorteile und Merkmale der Erfindung ergeben sich aus den übrigen Unterlagen. Die Erfindung wird im Folgenden anhand einer Zeichnung näher erläutert. Dabei zeigen:
- Figur 1: ein Abdecktuch gemäß der Erfindung mit unverformten Stabelementen;
- Figur 2: das Abdecktuch gemäß Figur 1 mit verformten Stabelementen und
- Figur 3: das Abdecktuch gemäß Figur 2 mit angeordnetem Referenzmittel.

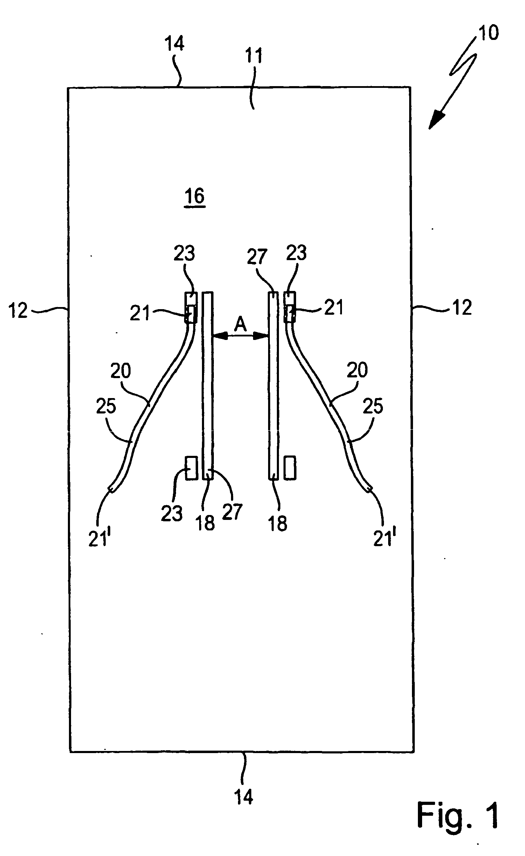

Figur 1 zeigt ein Abdecktuch, das in seiner Gesamtheit mit dem Bezugszeichen 10 versehen ist. Das Abdecktuch umfasst hierbei ein flächiges flexibles Abdeckelement 11, das eine rechteckige Gestaltung aufweist und insbesondere zwei längere Seitenkanten 12 sowie zwei kürzere Seitenkanten 14 aufweist, wobei die längere Seitenkanten 12 als Längskanten und die kürzeren Seitenkanten 14 als Querkanten bezeichnet werden sollen.

Das flexible und flächige Abdeckelement 11 weist dabei eine Fläche auf, die im Benutzungsfall einem Patienten zugewandt ist und in der Zeichnungsebene die untere Seite bilden würde, sowie eine vom Patienten abgewandte Seite, die mit dem Bezugszeichen 16 versehen ist und eine nach oben weisende Seite in der Abbildung darstellt. Das Abdeckelement 11 besteht hierbei aus einem transparenten Folienmaterial, insbesondere einer Polyethylenfolie.

Parallel zu den Seitenkanten 12 sind zwei Stabelemente 18 vorgesehen, die ebenfalls zueinander parallel verlaufen und über ihre gesamte Länge mit dem Abdecktuch unlösbar verbunden sind, zum Beispiel mittels Kleber fest fixiert sind. Der Abstand der Stabelemente 18 zueinander, hier mit dem Bezugszeichen A versehen, hängt von der Größe der benötigten Haube ab, also der Größe des Bereichs, der für ein aus der Ebene des Abdeckelements 11 herausragendes Referenzmittel eines Navigationssystems benötigt wird.

Darüber hinaus umfasst das Abdecktuch zwei Verkürzungsmittel, die mit dem Bezugszeichen 20 versehen sind, wobei je ein Verkürzungsmittel 20 einem Stabelement 18 zugeordnet ist und mit diesem zusammenwirkt. Die Verkürzungsmittel 20 sind dabei mit ihrem einen Endbereich 21 lösbar an einem zweiten Haftmittel 23 festgelegt. Das Verkürzungsmittel 20 ist dabei aus einem Flauschmaterial gebildet, das ein erstes Haftelement eines Haken- und Ösenhaftelementes ausbildet. Die ersten Haftmittel 25 erstrecken sich somit über die gesamte Länge und Fläche der Verkürzungsmittel 20.

Die zweiten Haftmittel 23 werden durch sogenannte Hakenhaftelemente eines Haken- und Ösenhaftmaterials gebildet und sind insbesondere im Bereich der Stabenden 27 angeordnet. Dabei befinden sich die zweiten Haftmittel 23 neben den Stabenden 27 und insbesondere parallel hierzu, sofern es sich um rechteckig ausgestaltete Haftmittel 23 handelt, sowie außerhalb des zwischen den Stabelementen 18 bestehenden Bereichs.

Wie bereits ausgeführt, ist das Verkürzungsmittel 20 mit einem seiner Endbereiche 21 an einem der zweiten Haftmittel 23 festgelegt. Das erste Ende 21 ist dabei lösbar an dem zweiten Haftmittel 23 festgelegt. Das zweite Ende 21' der Verkürzungsmittel kann dabei frei, also unverbunden mit den zweiten Haftmitteln 23, vorgesehen sein oder, sofern die Verkürzungsmittel 20 lang genug ausgebildet sind, ebenfalls mit den zweiten Haftmitteln 23 verbunden sein, ohne jedoch in der gezeigten Lagerungsposition vor Anwendung und Haubenbildung zu einer Verkürzung und Aufwölbung der Stabelemente 18 zu führen.

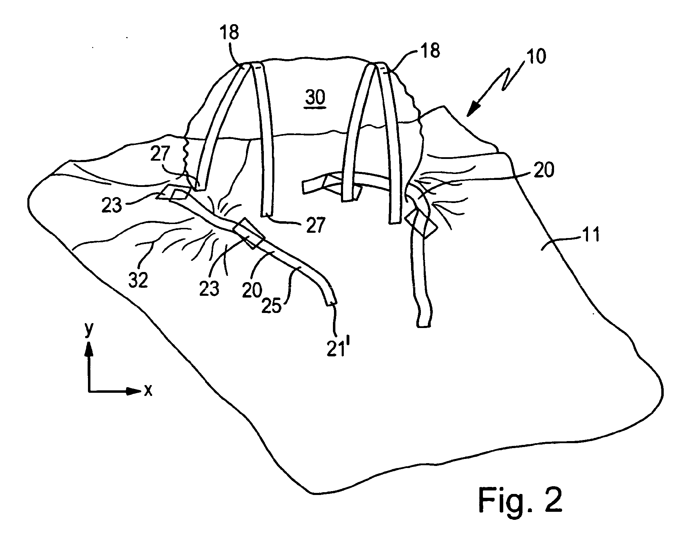

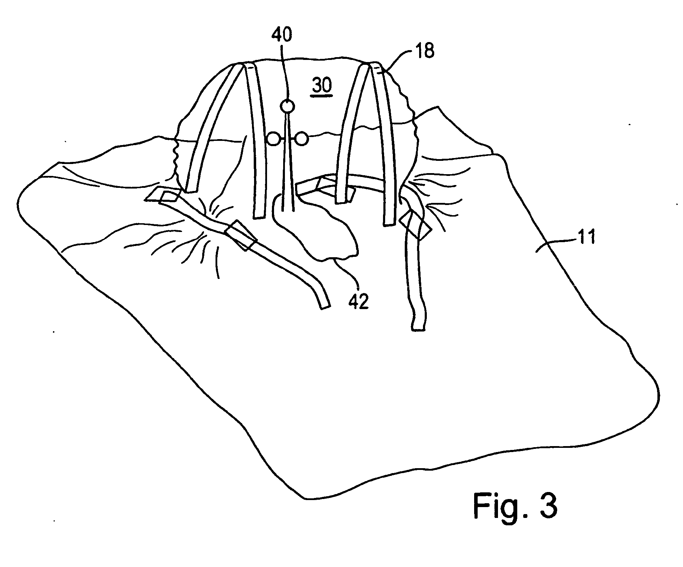

Figur 2 zeigt nun einen Anwendungsfall, bei dem ein Bereich 30 zur Aufnahme eines aus der Ebene des Abdeckelements 11 herausragenden Referenzmittels für ein Navigationssystem, das in Figur 3 gezeigt ist, aus der Ebene hervorragend gebildet wurde.

Dazu ist vorgesehen, dass beide Stabelemente 18 durch Krafteinwirkung bogenförmig verformt werden, so dass sich die Endbereiche 27 (Stabenden) der Stabelemente 18 einander in der Ebene des Abdeckelements 11 annähern und der Abstand zwischen den Stabenden 27 eines Stabelements 18 in der Ebene des Abdeckelements 11 verringert wird. Dies führt zu einem Aufwölben der Stabelemente 18 aus der Ebene, die hier mit X-Y bezeichnet ist, des Abdeckelements 11 heraus. Dadurch, dass die beiden stabförmigen Elemente 18 gleichermaßen aufgewölbt werden, bildet sich zwischen ihnen ein Bereich 30, der tunnelförmig ausgestaltet ist und der sich über die Ebene erhebt. Da die Stabelemente 18 und das Abdeckelement 11 fest miteinander verbunden sind, wird in diesem Bereich 30 auch das Abdeckelement 11 aus seiner ursprünglichen Ebene X-Y herausgehoben. Die Verkürzungsmittel 20 sind an beiden zweiten Haftelementen 23 je Stabelement 18 festgelegt. Außerhalb der Verkürzungsmittel 20 kommt es dabei zu einem Faltenwurf 32 des Materials des Abdeckelements 11. Innerhalb des Bereichs 30 ist jedoch eine faltenfreie Anordnung des Materials des Abdeckelements 11 vorgesehen, so dass es nicht zu einer Störung des Navigationssystems kommt.

Da die Verkürzungsmittel 20 über ihre gesamte Länge als Flauschmaterial ausgebildet sind und damit als erstes Haftelement 25, kann die Fixierung des Verkürzungselements an dem zweiten Haftelement in beliebigen Schritten erfolgen, je nachdem an welchem Punkt des Verkürzungsmittels dieses mit den zwei Haftelementen 23 verbunden wird. Je stärker die Verformung und damit die Aufwölbung des Stabelements 18, umso höher wird der dadurch gebildete Bereich 30 aus der Ebene herausgehoben. Auf der anderen Seite wird dabei die Ausdehnung des Bereichs 30 in der Ebene verringert.

Hier muss je nach Referenzsystem das bestmögliche Maß gefunden werden.

Figur 3 zeigt nun eine Gestaltung, die Figur 2 entspricht, wobei hier zusätzlich das Referenzmittel 40 gezeigt ist, das an einem Patienten festgelegt ist und sich aus einer Öffnung 42 am Patienten in den haubenartigen Bereich 30 des Abdeckelements 11 erstreckt.

Die Stabelemente 18 sind dabei plastisch verformbare Kunststoffstäbe, die über ihre gesamte Länge mit dem Abdeckelement 11 verbunden sind.

Besonders vorteilhaft ist an der vorliegenden Gestaltung, dass ein entsprechendes chirurgisches Abdecktuch 10 in einem ungebrauchten Zustand, wie ihn Figur 1 zeigt, keine Erstreckung außerhalb der Ebenenrichtung aufweist und so insbesondere auf ein Maß, das der Länge der Stabelemente 18 entspricht, gefaltet werden kann und so eine gute Lagerbarkeit und Verstaubarkeit gewährleistet. Darüber hinaus ist durch das geringere Lagervolumen auch eine einfache Sterilisierbarkeit gegeben.

Im Anwendungsfall kann dann einfach und schnell ein entsprechender Bereich 30, der auch als Haube bezeichnet sein kann, ausgebildet werden, wobei die Ausdehnung der Haube 30 durch die Verformung der Stabelemente und durch die Einstellung und/oder Stabilisierung mittels der Verkürzungsmittel 20 flexibel gewählt werden kann.

Darüber hinaus wird auf diese Weise eine faltenfreie Haube 30 gestaltet, so dass ein entsprechendes Navigationssystem, das insbesondere eine Kamera umfasst, das Referenzmittel 40 erfassen kann ohne durch Reflexionen gestört zu sein. Insbesondere handelt es sich bei dem Abdeckelement 11 um ein Folienmaterial, das so konzipiert ist, dass es infrarotdurchlässig ist. wird dabei intraoperativ das Ergebnis einer Operation beispielsweise mittels Computertomographen oder auch C-Bogen kontrolliert, so kann eine Navigation, wie sie heute vielfach üblich ist, mit einem System erfolgen, bei dem ein Kamerasystem das Referenzmittel 40 z. B. einen Referenzstern, der im geöffneten Körper des Patienten fixiert ist, verfolgen kann. Dabei soll auch während des Computertomographie- oder Röntgenvorgangs dieses Kamerasystem arbeiten können, um jede Veränderung oder Störung sofort zu erkennen.

## Patentansprüche

1. Chirurgisches Abdecktuch (10) umfassend ein flächiges, flexibles Abdeckelement (11) mit einem Bereich (30) zur Aufnahme eines aus der Ebene des Abdeckelements herausragenden Referenzmittels (40) für ein Navigationssystem, wobei der Bereich (30) mindestens zwei verformbare, längliche, mit dem Abdeckelement (11) zumindest partiell verbundene, jeweils zwei Stabenden (27) aufweisende Stabelemente (18) umfasst, welche den Bereich (30) zur Aufnahme des Referenzmittels (40) zwischen sich einschließen sowie mindestens ein an dem Abdeckelement (11) fixierbares, mit zumindest einem der Stabelemente (18) zusammenwirkendes längliches Verkürzungsmittel (20) zur Verringerung und/oder Fixierung des Abstandes der zwei Stabenden (27) zueinander in der Ebene des Abdeckelementes (11), so dass der Bereich (30) zur Aufnahme des Referenzmittels (40) sich aus der Ebene des Abdeckelements (11) erhebt.

2. Chirurgisches Abdecktuch nach Anspruch 1, wobei das Verkürzungsmittel (20) erste Haftmittel (25) aufweist, die mit zweiten Haftmitteln (23) am Abdeckelement (11) zusammenwirken.

3. Chirurgisches Abdecktuch nach Anspruch 2, wobei das Verkürzungsmittel (20) über seine gesamte Länge mit ersten Haftmitteln (25) ausgebildet ist.

4. Chirurgisches Abdecktuch nach Anspruch 2 oder 3, wobei mindestens zwei Haftmittel (23) je Verkürzungsmittel (20) vorgesehen sind.

5. Chirurgisches Abdecktuch nach einem der vorangehenden Ansprüche, wobei jedem der Stabelemente (18) mindestens ein Verkürzungsmittel (20) zugeordnet ist.

6. Chirurgisches Abdecktuch nach einem der vorangehenden Ansprüche, wobei die zwei Stabelemente (18) parallel zueinander angeordnet sind.

7. Chirurgisches Abdecktuch nach einem der vorangehenden Ansprüche 2-6, wobei die zweiten Haftmittel (23) wenigstens im Bereich der Stabenden (27) am Abdeckelement (11) vorgesehen sind.

8. Chirurgisches Abdecktuch nach einem der vorangehenden Ansprüche, wobei zumindest eines der Enden (21, 21') des oder der Verkürzungsmittel (20) lösbar mit dem Abdeckelement (11), insbesondere mit seinem ersten Haftmittel (25) mit dem zweiten Haftmittel (23) am Abdeckelement, verbunden ist.

9. Chirurgisches Abdecktuch nach einem der vorangehenden Ansprüche, wobei eines der Enden (21) des oder der Verkürzungsmittel (20) dauerhaft und unlösbar mit dem Abdeckelement (11) verbunden ist.

10. Chirurgisches Abdecktuch nach einem der vorangehenden Ansprüche 2 - 9, wobei das erste Haftmittel (25) ein Ösenhaftelement eines Haken- und Ösenhaftmaterials ist und das zweite Haftmittel (23) ein Hakenhaftelement eines Haken- und Ösenhaftmaterials ist.

11. Chirurgisches Abdecktuch nach einem der vorangehenden Ansprüche, wobei der Abstand der beiden Stabenden (27) eines Stabelements (18) kontinuierlich oder in vorgegebenen Positionen verkürzbar und fixierbar ist.

12. Chirurgisches Abdecktuch nach einem der vorangehenden Ansprüche, wobei der Bereich (30) des Abdeckelements (11), insbesondere das Abdeckelement (11) insgesamt, aus einem transluzenten, insbesondere transparenten Material, weiter insbesondere aus einer transluzenten, insbesondere einer transparenten Folie gebildet ist.

13. Chirurgisches Abdecktuch nach einem der vorangehenden Ansprüche, welches ein Neurochirurgisches Abdecktuch ist.

## Claims

1. Surgical drape (10) comprising a planar, flexible covering element (11) having an area (30) for accommodating a reference means protruding from the plance of the covering element (40) for a navigation system, wherein the area (30) comprises at least two deformable, elongated bar elements (18), which are at least partially connected to the covering element (11), each have two bear ends (27), which include the area (30) for accommodating the reference means (40) between themselves, and at least one elongated shortening means (20), which can be fixed on the covering element (11) and interacts with at least one of the bar elements (18), for reducing and/or fixing the distance of the two bar ends (27) from each other in the plane of the covering element (11) so that the area (30) for accommodating the reference means (40) is lifted out of the plane of the covering element (11).

2. Surgical drape according to the claim 1, wherein the shortening means (20) has first fastening means (25), which interact with second fastening means (23) on the covering element (11).

3. Surgical drape according to the claim 2, wherein the shortening means (20) is constituted to have first fastening means (25) over its entire length.

4. Surgical drape according to either one of the claims 2 and 3, wherein at least two fastening means (23) for each shortening means (20) are provided.

5. Surgical drape according to any one of the previous claims, wherein at least one shortening means (20) is allocated to each of the bar elements (18).

6. Surgical drape according to any one of the previous claims, wherein the two bar elements (18) are disposed parallel with each other.

7. Surgical drape according to any one of the previous claims 2 to 6" wherein the second fastening elements (23) are provided on the covering element (11) at least in the area of the bar ends (27).

8. Surgical drape according to any one of the previous claims, wherein at least one of the ends (21, 21') of the one or more shortening means (20) is detachably connected to the covering elements (11), in particular by its first fastening means (25) to the second fastening means (23) on the covering element.

9. Surgical drape according to any one of the previous claims, wherein one of the ends (21) of the one or more shortening means (20) is permanently and non-detachably connected to the covering element (11).

10. Surgical drape according to any one of the previous claims 2 to 9, wherein the first fastening means (25) is a loop fastening element of a hook-and-loop fastening material and the second fastening means (23) is a hook fastening element of a hook-and-loop fastening material.

11. Surgical drape according to any one of the previous claims, wherein the distance between the two bar ends (27) of a bar element (18) can be shortened and fixed continuously or in defined positions.

12. Surgical drape according to any one of the previous claims, wherein the area (30) of the covering element (11), in particular, the whole covering element (11), is constituted by a translucent, in particular, transparent material, further in particular, by a translucent, in particular, transparent film.

13. Surgical drape according to any one of the previous claims, which is a neurosurgical drape.

## Revendications

1. Drap chirurgical (10) comprenant un élément de couverture (11) flexible en nappe ayant une zone (30) de réception d'un moyen de référence (40) pour un système de navigation, qui fait saillie hors du plan dudit élément de couverture, ladite zone (30) comprenant au moins deux éléments en baguette (18) déformables oblongs qui sont reliés au moins en partie à l'élément de couverture (11) et présentent chacun deux extrémités de baguette (27) et qui enferment entre eux ladite zone (30) de réception du moyen de référence (40), ainsi qu'au moins un moyen de raccourcissement (20) oblong qui peut être fixé sur ledit élément de couverture (11), agit de concert avec au moins l'un des éléments en baguette (18) et est destiné à réduire et/ou à fixer la distance séparant les deux extrémités de baguette (27) l'une par rapport à l'autre dans le plan de l'élément de couverture (11), de sorte que la zone (30) de réception du moyen de référence (40) fait saillie hors du plan de l'élément de couverture (11).

2. Drap chirurgical selon la revendication 1, dans lequel ledit moyen de raccourcissement (20) comprend des premiers moyens adhésifs (25) qui coopèrent avec des seconds moyens adhésifs (23) sur ledit élément de couverture (11).

3. Drap chirurgical selon la revendication 2, dans lequel ledit moyen de raccourcissement (20) est réalisé sur l'ensemble de sa longueur avec des premiers moyens adhésifs (25).

4. Drap chirurgical selon la revendication 2 ou 3, dans lequel au moins deux moyens adhésifs (23) sont prévus par moyen de raccourcissement (20).

5. Drap chirurgical selon l'une quelconque des revendications précédentes, dans lequel au moins un moyen de raccourcissement (20) est associé à chacun des éléments en baguette (18).

6. Drap chirurgical selon l'une quelconque des revendications précédentes, dans lequel les deux éléments en baguette (18) sont disposés parallèlement l'un à l'autre.

7. Drap chirurgical selon l'une quelconque des revendications précédentes 2 à 6, dans lequel les seconds moyens adhésifs (23) sont prévus au moins au niveau des extrémités de baguette (27) sur ledit élément de couverture (11).

8. Drap chirurgical selon l'une quelconque des revendications précédentes, dans lequel au moins l'une des extrémités (21, 21') du ou des moyen(s) de raccourcissement (20) est reliée de manière amovible à l'élément de couverture (11), en particulier par son premier moyen adhésif (25) au second moyen adhésif (23) sur ledit élément de couverture.

9. Drap chirurgical selon l'une quelconque des revendications précédentes, dans lequel l'une des extrémités (21) du ou des moyen(s) de raccourcissement (20) est reliée à demeure et d'une manière inamovible audit élément de couverture (11).

10. Drap chirurgical selon l'une quelconque des revendications précédentes 2 à 9, dans lequel le premier moyen adhésif (25) est un élément adhésif à boucles d'une matière adhésive à crochets et à boucles, et le second moyen adhésif (23) est un élément adhésif à crochets d'une matière adhésive à crochets et à boucles.

11. Drap chirurgical selon l'une quelconque des revendications précédentes, dans lequel la distance séparant les deux extrémités de baguette (27) d'un élément en baguette (18) peut être raccourcie et fixée de façon continue ou dans des positions données.

12. Drap chirurgical selon l'une quelconque des revendications précédentes, dans lequel la zone (30) de l'élément de couverture (11), en particulier ledit élément de couverture (11) dans son ensemble, est réalisé(e) dans une matière translucide, en particulier transparente, encore en particulier dans une feuille translucide, en particulier transparente.

13. Drap chirurgical selon l'une quelconque des revendications précédentes, qui est un drap neurochirurgical.
